# EUROPEAN PATENT SPECIFICATION

(11) **EP 2 373 993 B1**
(45) Date of publication and mention of the grant of the patent: **05.08.2015**
(21) Application number: 09835551.4
(22) Date of filing: 14.12.2009
(51) Int. Cl.: G01N 33/50, A61K 31/10

(54) **METHODS FOR DETERMINING EFFICACY OF A THERAPEUTIC REGIMEN AGAINST DELETERIOUS EFFECTS OF CYTOTOXIC AGENTS IN HUMAN**
VERFAHREN ZUR BESTIMMUNG DER WIRKSAMKEIT EINER THERAPIE GEGEN DIE SCHÄDLICHE WIRKUNG VON ZYTOTOXIKA BEIM MENSCHEN
PROCÉDÉS DE DÉTERMINATION DE L'EFFICACITÉ D'UN RÉGIME THÉRAPEUTIQUE CONTRE LES EFFETS DÉLÉTÈRES D'AGENTS CYTOTOXIQUES CHEZ L'HOMME

(30) Priority: 22.01.2009 US 146391 P; 16.12.2008 US 122970 P
(43) Date of publication of application: 12.10.2011
(73) Proprietor: Onconova Therapeutics, Inc., Newtown, PA 18940 (US)
(72) Inventor: RAMESH, Kumar, Pennington NJ 08534 (US)
(74) Representative: Howard, Paul Nicholas
(86) International application number: PCT/US2009/067859
(87) International publication number: WO 2010/075026

(56) References cited:
- WO-A1-2005/108598
- WO-A2-2005/089268
- WO-A2-2005/089269
- US-A1- 2002 068 690
- US-A1- 2003 060 505
- US-A1- 2004 191 842
- US-A1- 2005 037 964
- US-A1- 2005 043 224
- US-A1- 2005 137 147
- MASTAN M ET AL: "PROTECTIVE EFFECT OF BACOPA MONNIERA L. ON CYTARABINE INDUCED BIOCHEMICAL CHANGES IN CHICK EMBRYO", INDIAN JOURNAL OF CLINICAL BIOCHEMISTRY, vol. 22, no. 1, 2007, pages 122-127, XP002682069,

## Description

### I. FIELD OF THE INVENTION

The present invention relates to methods for determining efficacy of a therapeutic regimen in human subjects exposed to or at risk of being exposed to a cytotoxic agent. The invention further relates to drug screening methods for identifying and evaluating efficiency of cytoprotective drugs in humans.

### II. BACKGROUND OF THE INVENTION

In order to achieve FDA approval for efficacy of a therapeutic regimen, the therapy must be applied to human subjects in clinical trials. In an effort to avoid clinical failures, there is an emphasis across the pharmaceutical industry on examining pharmacokinetic and safety profiles of drugs in human subjects earlier in the drug discovery process. It is often difficult to find human subjects willing to enter clinical trials specifically when a cytotoxic drug is used as part of the testing. Although oncology is more forgiving of delivery and side effect complications than most therapeutic areas, cancer discovery programs are not immune from this trend. It is also unethical to subject normal human subjects to clinical trials that require the use of cytotoxic agents such as ionizing radiation.

Two major concerns in drug development are the need to predict the efficacy and safety of a potential drug candidate before clinical trials are initiated and how to predict which cases are going to respond to a particular treatment. During the development process therefore, drug candidates are typically batch tested on a variety of cell lines. Those that seem to have the desired effect on the cell lines are then tested in animals during the preclinical phase. Neither process, however, replicates the true clinical situation.

In particular, there is a need to develop radioprotective agents and therapies that can deal with ionizing radiation and specifically address the aftermath of an act of urban nuclear terrorism. In the recent years, the US government has committed hundreds of millions of dollars to projects dealing with ionizing radiation and dealing with the aftermath of an act of urban nuclear terrorism. The body's most susceptible vital tissue to radiation is the bone marrow, specifically the hematopoietic stem cells that give rise to new blood cells. Stems cells are impaired at doses as low as half a gray of radiation and are usually destroyed completely and permanently above 5 grays. When stem cells die, blood-cell counts, most critically those of neutrophils and platelets, start to drop, eventually plunging to zero after days or weeks. Without neutrophils, the first-responders of the immune system, radiation victims are at high risk of opportunistic infections and without platelets blood cannot clot, leading to potentially fatal bleeding from even the smallest wound. Upwards of 5 grays, the gastrointestinal tract is also affected. Radiation kills any rapidly dividing cells, such as those lining the intestinal tract. The resulting damage can cause bacteria from the intestine to leak into the bloodstream, where they overwhelm the already compromised immune system and cause septic shock. At exposures above 10 grays, the central nervous system is damaged too, and death is certain, with or without treatment.

In 2007, the U.S. government provided incentive to companies to develop drugs that preserve and restore neutrophil counts in a radiation syndrome, with secondary emphasis on platelets. The government's mission is to gain a better understanding of the biology of radiation damage, find faster ways of diagnosing radiation exposure levels, and discover better drugs. So far no such drugs have been approved in the U.S., but there are a few candidates in the pipeline.

To overcome these deficiencies, there is a need for high throughput assays for screening large numbers of potential drug candidates produced during the discovery phase of drug development and allow those demonstrating cytotoxic protective promise to continue further in research and development. To address this need, *in vitro* cell-based assays that demonstrate a high degree of predictability during different phases of drug development have been developed. In order to render cell-based assays more predictive of clinical settings, investigators have used primary cells exhibiting a high degree of predictability that coupled with a high-throughput component, could have a significant impact and value on the drug development process.

There is therefore an intense interest in agents that are well-tolerated by the subject exposed to cytotoxicity, and are of high potency in effecting stimulation of stem cell and/or progenitor cell recruitment. Stem cells have the ability to divide for indefinite periods in culture and to give rise to specialized cells. Typically, stem cells are divided into two main groups: adult stem cells and embryonic stem cells. Stem cells may also be generated through artificial means such as nuclear transfer, cytoplasmic transfer, cell fusion, parthenogenesis and reprogramming. Isolated stem cells can give rise to many types of differentiated cells, and can be used to treat many types of diseases.

Adult stem cells are undifferentiated but are present in differentiated tissues, and are capable of differentiation into the cell types from the tissue that the adult stem cell originated. Adult stem cells have been derived from various sources, such as the nervous system (McKay, 1997, Science 276:66-71; Shihabuddin, et al., 1999, Mol. Med Today 5:474-480); bone marrow (Pittenger, et al., 1999, Science 284:143-147; Pittenger, M. F. and Marshak, D. R. (2001) In: Mesenchymal stem cells of human adult bone marrow. Marshak, D. R., Gardner, D. K., and Gottlieb, D. eds. (Cold Spring Harbor, N.Y.: Cold Spring Harbor Laboratory Press) 349-374); adipose tissue *(*Gronthos, et al., 2001, J. Cell. Physiol. 189:54-63), dermis (Toma, et al., 2001, Nature Cell Biol. 3:778-784); pancreas and liver (Deutsch, et al., 2001, Development 128:871-881). Stem cells have also been isolated from umbilical cord (Rogers, et al., 2004, Best Pract Res Clin Obstet Gynaecol. 18(6):893-908; Wang et al., 2004, Stem Cells 22(7):1330-1337; Surbek, et al, 2002, Ther Umsch. 59(11):577-582; and placenta (Yen et al., 2005, Stem Cells 23(1):3-9). Stem cells of the adult type are also found in smooth muscle tissue, striated muscle tissue, cardiac muscle tissue, bone tissue, bone spongy tissue, cartilage tissue, pancreatic ductal tissue, spleen tissue, thymus tissue, tonsil tissue, Peyer's patch tissue, lymph nodes tissue, thyroid tissue, epidermis tissue, dermis tissue, subcutaneous tissue, heart tissue, lung tissue, vascular tissue, endothelial tissue, blood cells, bladder tissue, kidney tissue, digestive tract tissue, esophagus tissue, stomach tissue, small intestine tissue, large intestine tissue, adipose tissue, uterus tissue, eye tissue, lung tissue, testicular tissue, ovarian tissue, prostate tissue, connective tissue, endocrine tissue, and mesentery tissue.

Several patents disclose various aspects of adult stem cells. For example, U.S. Pat. No. 5,556,783 discloses methods of culturing hair follicle stem cells, while U.S. Pat. No. 5,486,359 discloses methods of isolating human mesenchymal stem cells. U.S. Pat. Nos. 4,714,680, 5,061,620, and 5,087,570 provide examples of hematopoietic stem cells. U.S. Pat. No. 6,242,252 provides examples of liver stem cells. Hematopoietic cells have very important roles in a number of different processes in the body. For example, leukocytic hematopoietic cells are important in maintaining the body's defenses against disease; monocytes, macrophages and lymphocytes are involved in potentiating the body's responses to infection and tumors, while granulocytes are involved in overcoming infection, parasites and tumors. Platelets, another hematopoietic cell, form an important element in the hemostatic mechanism through initiating thrombus formation by their adhesion to each other and to damaged surfaces, and by the release to factors which assist in the formation of the fibrin clot. Erythrocytes are mainly involved in the transport of oxygen. All of these blood cells are derived from a single progenitor cell called the hematopoietic stem cell.

Hematopoietic stem cells make up only a small percentage of bone marrow cells and are normally quiescent. However, when stimulated to divide, these stem cells produce a differentiated daughter cell with great proliferative potential. Sequential rounds of division and differentiation give rise to an enormous amplification of cell numbers which is necessary for the production of mature blood cells. This process of division and differentiation is subject to regulation at many levels to control cell production.

Cytotoxicity and/or cytoprotectivity drug testing is difficult to test in human subjects because of the potential deleterious effects that results from such tests. Biological assays have been developed to determine effectiveness of a cytoprotective drug *in vitro* using specific biomarkers to detect cytotoxicity resulted from exposure to toxic agents, adverse side-effect of a medication for a given clinical setting and at a specific dosage, radiological, biological, or chemical warfare agents, and other hazardous materials. Numerous studies have led to the definition of functions of several hematopoietic regulatory messengers biomarkers that are abnormally expressed (e.g., down-regulated, up-regulated, mutated, etc) and their identification and isolation for cytotoxic testing. These biomolecules have been characterized as stimulatory, *e.g*., Colony Stimulating Factors (CSFs) and interleukins (IL-1, IL-3, IL-5, IL-8 and IL-9); inhibitory, *e.g*., transforming growth factor-β (TGF- β), interferon, prostaglandin E, tumor necrosis factor, macrophage inflammatory protein-1 (MIP-1), lactoferrin, acidic isoferritins, AcSKDP, and pEEDCK (a synthetic HP5B monomer); or enhancing, *e.g.,* TGF β, IL-6, IL-4, IL-9, IL-11, MIP-1., MIP-2, leukemia inhibitory factor and Steel factor. Pelus et al. Experimental Hematology 1994, 22:239-247. Stimulatory biomolecules have been found to promote division of particular cell lineages. For example, G-CSF derives neutrophil production, while erythropoietin promotes formation of erythrocytes.

Recruitment of stem cells and/or progenitor cells is important in a variety of assay applications. Vasculogenesis, which involves the growth of vessels derived from endothelial progenitor cells, is an example of such a process. Vasculogenesis, as well as angiogenesis, the process by which new blood vessels are formed from extant capillaries, and the factors that regulate these processes, are important in embryonic development, inflammation, and wound healing, and also contribute to pathologic conditions such as tumor growth, diabetic retinopathy, rheumatoid arthritis, and chronic inflammatory diseases (see, *e.g.,* U.S. Pat. No. 5,318,957; Yancopoulos et al. (1998) Cell 93:661-4; Folkman et al. (1996) Cell 87;1153-5; and Hanahan et al. (1996) Cell 86:353-64).

Several angiogenic and/or vasculoaenic agents with different properties and mechanisms of action are well known in the art. For example, acidic and basic fibroblast growth factor (FGF), transforming growth factor alpha (TGF-α) and beta (TGFβ), tumor necrosis factor (TNF), platelet-derived growth factor (PDGF), vascular endothelial cell growth factor (VEGF), and angiogenin are potent and well-characterized angiogenesis-promoting agents. In addition, both nitric oxide and prostaglandin (a prostacyclin agonist) have been shown to be mediators of various angiogenic growth factors, such as VEGF and bFGF. However, the therapeutic applicability of some of these compounds, especially as systemic agents, is limited by their potent pleiotropic effects on various cell types *in vitro.*

There is a lack or insufficient cytotoxic protective therapies and cytotoxic protective drugs today and there is a continuing need to identify and develop non-toxic and effective radioprotectors and cytotoxic protective therapies. One reason for this shortage, as explained above, is the difficulty of testing these compounds in human subjects. FDA rules presently allow efficacy and biopharmacokinetic determination in animal models to be extrapolated to humans. However, extrapolation of data from such animal models to humans may not accurately identify pharmacokinetic parameters and address efficacy of the therapy in humans. The drug screening and efficacy determination of therapies according to the present invention satisfies these and other long felt needs by providing a platform to analyze and determine the effects of therapies used to prevent cytotoxicity to humans exposed to or at risk of being exposed to cytotoxicity.

### III. SUMMARY OF THE INVENTION

The present invention provides drug screening and therapeutic methods for screening for and determining efficacy of a therapeutic regimen to protect a subject from deleterious effects of a cytotoxic agent.
In one aspect, the invention provides a method for determining efficacy of a therapeutic regimen to protect a human subject from deleterious effects of a cytotoxic agent comprising i) exposing a) target cells extracted from a subject prior to treatment with a cytotoxic protective drug, and b) target cells extracted from the subject after treatment with the cytotoxic protective drug, to the cytotoxic agent; and ii) analyzing and comparing one or more parameters indicative of viability and growth conditions in target cells of step i), wherein a favorable viability and growth condition of the target cells of step ib) as compared to target cells of step ia) is indicative of the efficacy of the therapeutic regimen in the subject.

The administration of the cytotoxic protective drug to the subject may be an *in vivo* administration. The *in vivo* administration comprises direct administration of the cytotoxic protective compound to the subject concomitantly, or before or after administration of the cytotoxic agent. Alternatively, the *in vivo* administration is accompanied by *ex-vivo* administration of the cytotoxic protective drug, wherein the *ex* vivo administration comprises transplantation of *ex-vivo* cells that have previously received an effective concentration of the cytotoxic protective drug.

The direct *in vivo* administration of the cytotoxic protective drug is through a wide variety of routes, excluding topical (including buccal and sublingual) administration but including transdermal, intraperitoneal, intracranial, intracerebroventricular, intracerebral, intravaginal, intrauterine, oral, rectal, ophthalmic (including intravitreal or intracameral), nasal, parenteral (including subcutaneous, intraperitoneal, intramuscular, intravenous, intradermal, intracranial, intratracheal, and epidural), with the proviso that no direct topical administration should be performed. The direct *in vivo* administration of the cytotoxic protective drug through topical routes (including buccal and sublingual) is expressly disclaimed herein.

The cytotoxic protective drug may also be administered to a subject *in vivo, in vitro,* or both before, during, or after exposure to the cytotoxic agent. The direct exposure to or administration of a cytotoxic agent to a mammal or human subject is expressly disclaimed herein.

The target cells within the scope of the invention comprise cells that are directly, indirectly, or specifically affected by the cytotoxic agent upon exposure. These cells include epithelial tissues, connective tissues (blood, bone, cartilage), muscle tissues, nerve tissues, stem tissues and cells, progenitor cells, and subclasses and categories of these tissues and cells.

In one embodiment, the target cells comprise stem cells from the hematopoietic system, epithelial mucosa of the gastrointestinal tract, the dermis of the skin, the germ cells of the reproductive organs, the epithelium of the eye cornea, or a combination thereof. The stem cell from the hematopoietic system comprises stem cell and progenitor cells from bone marrow.

The cytotoxic agent comprises a chemical cytotoxic agent, a radiological cytotoxic agent, an ionizing radiation, a biological cytotoxic agent, a biological warfare agent, a chemical warfare agent, a drug with adverse side effects in humans, or a combination thereof.

In one embodiment, the cytotoxic protective drug comprises small molecule compounds, peptides, gene expression products, chemoprotective drugs, radioprotective drugs, antiviral or antibacterial drugs, angiogenic or ant-angiogenic drugs, or anti-inflammatory drugs.

In another embodiment, the radioprotective drug exhibits a synergistic anti-cell proliferation activity in tumor cells of cancer patients when administered with chemotherapeutic agents or radiation.

In yet another embodiment, radioprotective drug is a non-toxic radioprotective drug comprising compounds of α, β unsaturated aryl sulfones including E-4-Carboxystyryl-4-chlorobenzylsulfone sodium salt.

The radioprotective drug exerts its activity through a wide variety of cellular and biochemical mechanisms and pathways including, by way of example and not limitation, inhibiting the activities of radiation-sensitive genes, inhibition of apoptosis, and protecting DNA from radiation- induced damage, or a combination thereof.

The viability and growth condition of the target cells are determined by analyzing one or more parameters or biomarkers that are up-regulated, down-regulated, or abnormally expressed in the target cells upon exposure to the cytotoxic agent. The biomarkers include nucleic acid or peptide or protein molecules related to intracellular pathways involved in apoptosis, cell cycle arrest or repair, proliferation, senescence, or differentiation of target cells, modulating activity of genes controlling one or more cellular responses, gene expression products, enzymes and products of specific biochemical pathways and protein cascades, cell receptors, pre-apoptosis signaling pathways and molecules, antigen or antibody fragments, agonists or antagonists of peptides, cytogenetics markers, or a combination thereof.

In one embodiment, the intracellular pathways comprise hematopoietic regulatory pathways and the one or more parameters comprise hematopoietic regulatory messenger biomarkers.

In another embodiment, intracellular pathways comprise one or more pathways of the mitogen activated protein kinase superfamily.
In another aspect, the invention provides for a method of screening for cytotoxic protective drug comprising: i)exposing a) target cells extracted from a non-human subject prior to treatment with a cytotoxic protective test compound, and b) target cells extracted from the non-human subject after treatment with the cytotoxic protective test compound, to the cytotoxic agent; and ii) analyzing and comparing one or more parameters indicative of viability and growth conditions in target cells of step i), wherein a favorable viability and growth condition of the target cells of step ib) as compared to target cells of step ia) is indicative of the potential of the cytotoxic protective test compound for development as cytotoxic protective drug.

In one embodiment, the drug screening method additionally determines the pharmacodynamics of the cytotoxic protective drug comprising determination of dosage, time of administration, time interval between administration of cytotoxic and cytotoxic preventive drug, frequency of administration, and sequence of administration of cytotoxic protective drug and cytotoxic agent, bioavailability of the cytotoxic protective drug, or a combination thereof.

In yet another embodiment, the cytotoxic protective compound comprises small molecule compounds, peptides, gene expression products, chemoprotective drugs, radioprotective drugs, antiviral or antibacterial drugs, angiogenic or anti-angiogenic drugs, or anti-inflammatory drugs, among others.

Also disclosed is an *ex-vivo* therapeutic regimen for protecting subjects from cytotoxity of radiation therapy comprising (i) removing a portion of the subject's bone marrow cells; (ii) administering an effective amount of at least one radioprotective α, β unsaturated aryl sulfones comprising E-4-Carboxystyryl-4-chlorobenzylsulfone sodium to the subject; (iii) irradiating the bone marrow cells with an effective amount of an ionizing radiation; iv) implanting the radiated bone marrow cells into the subject. The bone marrow cells comprise stem cells and/or progenitor cells.

The cytotoxic protective drug may be E-4-Carboxystyryl-4-chlorobenzylsulfone sodium and it is administered prior to, during and/or after reimplantation of the bone marrow cells.

The cytotoxic protective drug may be administered in a therapeutically effective amount to a subject prior to, during, and/or after the exposure to cytotoxicity. For example, the cytotoxic protective drug may be administered at least a few hours or at most one week prior to, or after exposure to cytotoxic agent. For example, the cytotoxic protective drug is administered about one week, 72- 48 hrs, 24-18 hrs, 12-6 hrs, or less prior to or after exposure to cytotoxicity, or both.

It is intended herein that by recitation of such specified ranges, the ranges recited also include all those specific integer amounts between the recited ranges. For example, in the range about 72-48 hrs, it is intended to also encompass 71-65, 64-55-, 54-49, etc, without actually reciting each specific range therewith.

The cytotoxic protective drug (*e.g*. the radioprotective or chemoprotective drug) may be administered to a cancer patient prior to the exposure to chemotherapy or radiotherapy in a therapeutically effective concentration to exert a cytotoxic protective effect on the normal cells. The cytotoxic protective drug is administered, for example, about 24 hours, 18 hours, 12 hours, or 6 hours prior to administration of the chemotherapy or radiation.

As disclosed herein, the *ex vivo* therapeutic regimen may result in destruction of malignant cells or reduction the number of malignant cells in bone marrow of the subject.

In yet another aspect, the disclosure provides a test kit for screening candidate cytotoxic protective drugs or determining efficacy of cytotoxic protective drug comprising solutions and devices for extraction of target cells from the subject, one or more test compounds as potential cytotoxic protective drugs, cell and tissue culture media and plates, one or more cytotoxic agents, biomarkers and assays to analyze parameters indicative of viability and growth conditions in target cells, one or more vessels containing the necessary reagent mixes and instructions for the use thereof for performing the assays. In an embodiment, the invention provides the use of the test kit in the claimed methods of the invention.

These and other aspects, features and advantages of the present invention will become apparent after reading the following detailed description of the disclosed embodiments and the appended claims.

### VI. BRIEF DESCRIPTION OF THE FIGURES

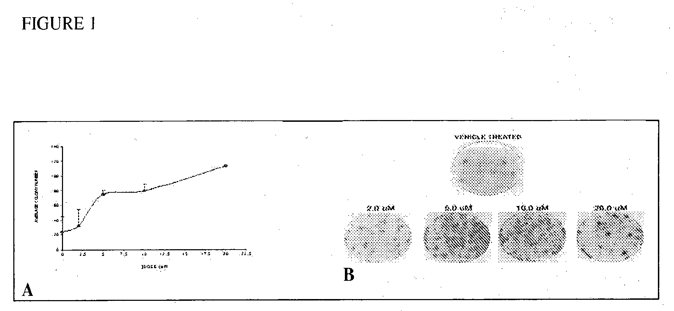

**FIGURE 1****.** Protection of HFL-1 cells against Ionizing Radiation (IR) by ON 01210.Na. Following drug treatment and radiation exposure, cells were re-plated and the number of colonies from each plate was enumerated three weeks later. A. Relation of concentration of ON 01210.Na to number of surviving colonies. B. Stained clonogenic assay plates.

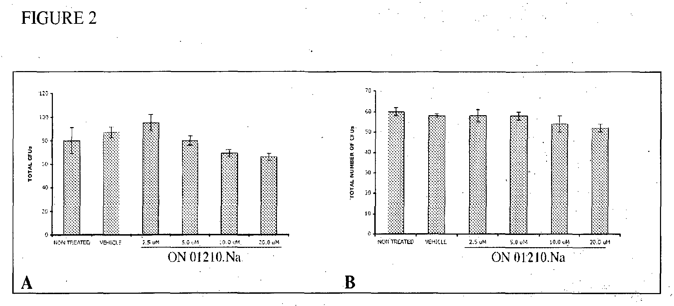

**FIGURE 2****.** ON 01210.Na is not cytotoxic to human bone marrow cells *in vitro.* Human bone marrow cells were treated with various concentrations of ON 01210.Na for (A) 2 hrs or (B) 24 hrs. The cells were then plated into methocult medium. Total colony forming units were determined 14 days later. ON 01210.Na did not interfere with the growth or differentiation of human bone marrow cells even after long-term exposure at high concentrations.

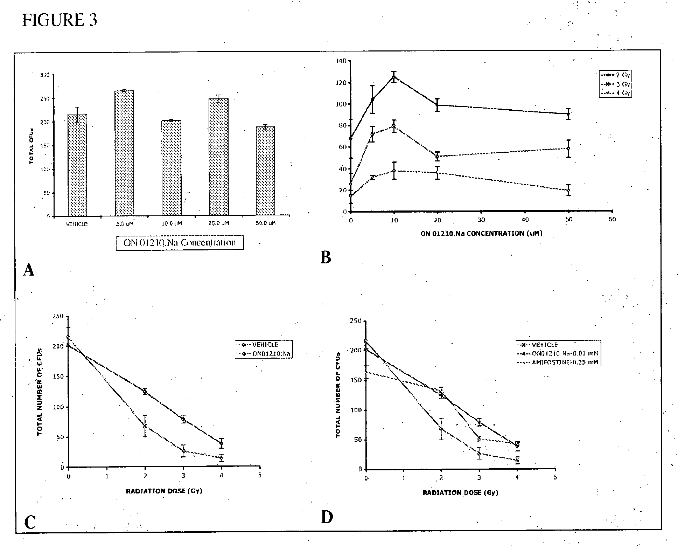

**FIGURE 3****.** *In vitro* radiation protection of human bone marrow cells. Human bone marrow cells were treated with increasing concentrations of ON 01210.Na for 3 hours, and then irradiated with increasing doses of ionizing radiation. The cells were plated into methocult and the total number of CFUs was determined 14 days later. ON 01210.a treatment without radiation was non-toxic at all concentrations tested (A). ON 01210.Na protected human bone marrow cells in a dose responsive manner (B). When the survival curves were plotted at the optimal ON 01210.Na concentration (10 uM), ON 01210.Na (C) had a DRF value of 1.6 while the optimal dose of amifostine (0.25 mM) had a DRF value of 1.5.

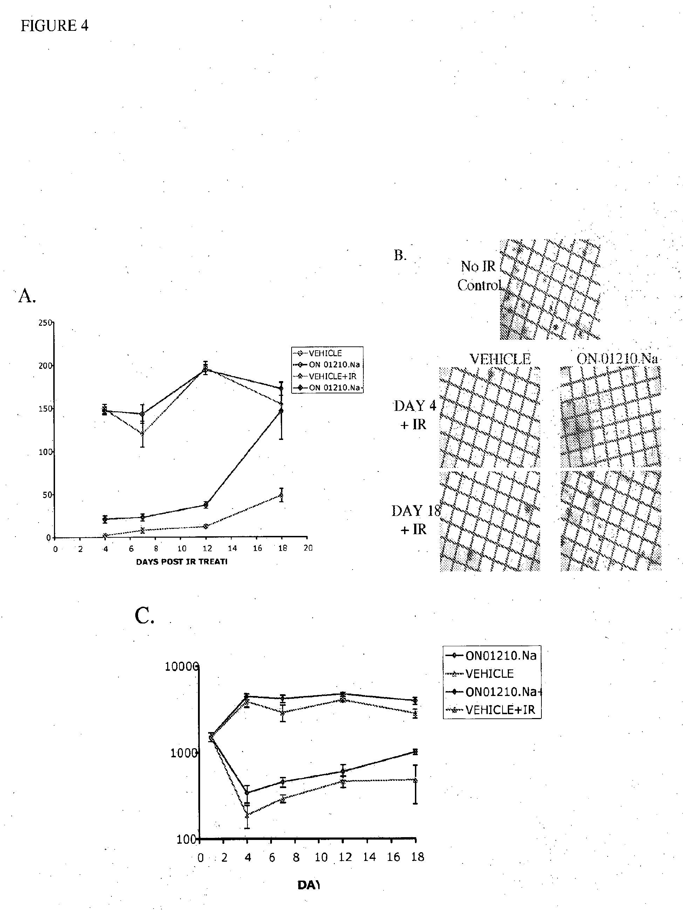

**FIGURE 4****.** ON 01210.Na protects the bone marrow of irradiated mice. A. Mice were injected with 500 mg/kg of ON 01210.Na formulated in 1% Polysorbate 80/K-phosphate buffer or vehicle alone by sc injections minus 24 hrs and minus 15 minutes before 5.5 Gy of whole body ionizing radiation treatment. Whole blood was collected on the indicted days and WBC counts were performed following RBC lysis. The data is plotted as the average of three mice per group +/-SEM (N:3x3=9 plates). B. Pictures of representative plates. ON 01210.Na protected the bone marrow from complete ablation of stem cells and significantly increased the recovery of the bone marrow. C. White blood cell counts. Whole blood was collected on the indicted days and WBC counts were performed following RBC lysis. The data is plotted as the average of 3 mice per group ±SEM (N=3).

### DETAILED DESCRIPTION OF THE INVENTION

### Definitions

As used herein, "cytotoxic agents" are broadly defined and encompass agents and elements that demonstrate toxicity on some or all cells or tissues. Cytotoxic agents within the scope of this invention encompass a wide variety of chemical, physical and biological substances that cause harm to human at certain concentration, dosage, degrees or magnitudes. The cytotoxic agents include, by way of example and not limitation, general classes of pharmaceutical drugs that are used to treat a disease or disorder with adverse side effects and toxicity on normal tissues. In one embodiment, the cytotoxic drugs include cancer-related drugs including, chemotherapeutic agents, radiotherapeutic agents, antiproliferative agents, anti-angiogenic agents, or non-cancer drugs. Cytotoxic agents within the scope of the invention also include ionizing radiation, agents of biological or chemical warfare agents, agents or elements caused by exposure to extreme physical and/or environmental conditions that may be used to train military personnel or astronauts, such as high intensity light, radiation, ozone, ultrasound, turbulence, extreme high or low temperature, etc.

As used herein, "subjects" are broadly defined as animals and humans. The term "animal" as used herein refers to any vertebrate animal specifically mammals including, but not limited to, bovine, ovine, porcine, equine, canine, feline species, non-human primates including apes and monkeys, rodents such as rat and mouse, and lagomorphs such as rabbit and hare. In one embodiment, the subjects are healthy individuals who have been subjected to cytotoxicity or who are at risk for incurring or exposure to cytotoxicy. In another embodiment, the subjects are asymptomatic or symptomatic patients exposed to or at risk of being exposed to drugs or radiation with cytotoxicity. In yet another embodiment, the subjects are military or private personnel who are exposed to or at risk of being exposed to cytotoxic agents and elements including ionizing radiation, biological warfare agents, chemical warfare agents or cytotoxicity caused by adverse environmental conditions, such as extreme temperature, cold, agitation, turbulence, etc.

As used herein "cytotoxic protective drug," includes small molecule compounds, biological molecules and substances of diverse structure and function. Cytotoxic protective drugs may be polypeptide or nucleotide-based molecules or non-polypeptide or nucleotide-based molecules, including small molecules, carbohydrates, lipids, or a combination thereof. The polypeptide and nucleotide-based molecules include any protein, polypeptide or peptide fragment, or biologically active fragments thereof produced in the course of the transcription, reverse-transcription, polymerization, translation, post-translation and/or expression of a nucleic acid molecule. Also encompassed within the scope of the cytotoxic protective drugs are substantially homologous polypeptides, oligopeptides, homodimers, heterodimers, variants of the polypeptides, modified polypeptides, derivatives, analogs, fusion proteins, agonists, antagonists, or antibody of the polypeptides, among others, derived from natural, recombinant, and/or synthetic sources. The nucleic acid molecules include cDNA, RNA, DNA/RNA hybrid, anti-sense RNA, mRNA, iRNA, Si RNA, ribozyme, and genomic DNA, among others.

As used herein "small molecule compounds" include, but are not limited to, carbohydrates, carbohydratemimetics, peptidomimetics, organic or inorganic compounds *(e.g.* including heteroorganic and organometallic compounds) and salts, esters, and other pharmaceutically acceptable forms of such compounds. In a preferred embodiment, the small molecule compounds of the invention comprise compounds in the family of α, β unsaturated aryl sulfones including (E)-Styrylbenzylsulfones (e.g., E-4-Carboxystyryl-4-chlorobenzylsulfone sodium salt (ON 01210.Na,)).

As used herein "biologically active fragments" refer to fragments exhibiting activity similar, but not necessarily identical, to an activity of the cytotoxic protective drug of the present invention. The biologically active fragments may have improved desired activity, or a decreased undesirable activity.

As used herein "polypeptides" include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types.

As used herein," side effect" refers to an unintended occurrence, whether adverse or beneficial, that results from taking a cytotoxic agent or a drug. Side effects refer to problems that occur when treatment goes beyond the desired effect, or problems that occur in addition to the desired therapeutic effect and they are usually unwanted and hazardous although sometime they may be beneficial. The common side effects of cancer treatment is the apoptosis of normal cells which causes severe fatigue, nausea, vomiting, decreased blood cell counts, hair loss, and mouth sores, etc.

As used herein, "tissue" refers to a group or collection of similar cells and their intercellular matrix that act together in the performance of a particular function. The primary tissues are epithelial, connective (including blood), skeletal, muscular, glandular and nervous.

As used herein, "target cell" refers to any cell population that may derive from normal or malignant epithelial, connective, skeletal, muscular, glandular and nervous tissues or a combination thereof. Especially, target cells are derived from hematopoietic stem and progenitor cells, or subpopulations thereof. Target cell also refer to any cell population of a solid or non-solid tissue including, but not limited to, a peripheral blood cell population, hematopoietic cells, including bone marrow, umbilical cord blood, fetal liver cells, yolk sac and differentiating embryonic stem cells or differentiating primordial germ cells or embryonic germ cells. The cells may also be a primary cell line population including, but not limited to, a leukemic cell line. Examples of leukemic cell lines include, but are not limited to, an acute lymphocytic leukemia, an acute myeloid leukemia, a chronic lymphocytic leukemia, a chronic myeloid leukemia and a pre-B acute lymphocytic leukemia, acute myelogenous leukemia, acute T-cell leukemia, acute lymphoblastic leukemia, chronic myeloid leukemia, acute monocytic leukemia and B-cell leukemia.

As used herein, "cell line" refers to cells that are harvested from a human or animal adult or fetal tissue, including blood and cultured *in vitro,* including primary cell lines, finite cell lines, continuous cell lines, and transformed cell lines.

As used herein, "cell lineage" refers to a cell line derived from a stem cell or progenitor cell that is committed to producing a specific functional cell including, but not limited to, mature cells of the hematopoietic system.

As used herein, "primary cells" refer to cells obtained directly from a human or animal adult or fetal tissue, including blood. The "primary cells" or "cell lines" may also be derived from a solid tumor or tissue that may or may not include a hematopoietic cell population, and can be suspended in a support medium. The primary cells may comprise a primary cell line.

As used herein, "hematopoietic stem cells" refer to pluripotent stem cells or lymphoid or myeloid (derived from bone marrow) stem cells that, upon exposure to an appropriate cytokine or plurality of cytokines, may either differentiate into a progenitor cell of a lymphoid or myeloid cell lineage or proliferate as a stem cell population without further differentiation having been initiated. "Hematopoietic stem cells" include, but are not limited to, colony-forming cell-blast (CFC-blast), high proliferative potential colony forming cell (MT-CPC), colony-forming unit-granulocyte, erythroid, macrophage, and megakaryocyte (CFU-GEMM) cells, among others.

As used herein, "progenitor" and "progenitor cell" refer to primitive hematopoietic cells that have differentiated to a developmental stage that, when the cells are further exposed to a cytokine or a group of cytokines, will differentiate further to a hematopoietic cell lineage. "Progenitors" and "progenitor cells" as used herein also include "precursor" cells that are derived from some types of progenitor cells and are the immediate precursor cells of some mature differentiated hematopoietic cells. The terms "progenitor", and "progenitor cell" as used herein include, but are not limited to, granulocyte-macrophage colony-forming cell (GM-CFC), megakaryocyte colony-forming cell (Mk-CFC), burst-forming unit erythroid (BFU-E), B cell colony-forming cell (B-CFC) and T cell colony-forming cell (T-CFC). "Precursor cells" include, but are not limited to, colony-forming unit-erythroid (CFU-E), granulocyte colony forming cell (G-CFC), colony-forming cell-basophil (CFC-Bas), colony-forming cell-eosinophil (CPC-Bo) and macrophage colony-forming cell (M-CFC) cells.

As used herein, "cell cycle" refers to the cycle of stages in the replication of eukaryotic cell. The cycle comprises the four stages G1, S, G2 and M, wherein the S-phase is that portion of the cycle wherein the nucleic acid of the cell is replicated. Thus, a cell identified as being in the S-phase of the cell cycle is also identified as being a proliferating cell. As used herein, "modulating proliferation" refers to the ability of a compound to alter the proliferation rate of a population of cells in general and vascular, hematopoietic stem or progenitor cells in particular. A compound may be toxic, wherein the proliferation of the cells is abnormally slowed, halted, or enhanced such as, for example, by the addition to the cells of a cytokine or growth factor.

The invention as disclosed and described herein relates generally to methods of determining efficacy of a therapeutic regimen for protection of mammals and especially human subjects from deleterious effects of cytotoxicity. The invention further provides combination of cell-based and *in vivo* assays and/or *ex-vivo* assays for both the identification of new potential cytoprotective compounds and/or optimization of lead known cytoprotective compounds to protect against cytotoxicity.

Also encompassed within the scope of the invention are methods and uses of kits to determine pharmacokinetics determination of the cytotoxic protective drug. These methods are effective in optimization of a cytoprotective compounds to achieve maximum efficacy against the effects of a cytotoxic agent. For example, determination of dosage of a cytoprotective drug, its time of administration, bioavailability of cytotoxic protective drugs, and general analysis of effectiveness of these drugs can be tested without the need of administering the cytotoxic agent to the subjects in a clinical trial setting. In this way, the uses of the kits and methods of the present invention reduce the likelihood of potentially harmful side-effects of a known drug or other types of cytotxicity.

In one aspect, the invention provides drug screening methods using combination of *in vitro, ex vivo* assays using stem or progenitor cells that provide the required predictability and sensitivity because they allow detection of any effect or insult by the test compounds before it is observed in the patient's blood circulation. The invention provides methods and strategies to test the differential effect of drugs on the various cells and tissues and, in particular, on lympho-hematopoietic lineages with sufficient predictability and sensitivity without reliance on the end stage blood parameters. *In vivo,* an insult at the stem or early progenitor cell level requires a certain amount of time for the effect to be detected at the peripheral blood level and by the time the effect is observed, adverse reactions by the patient are likely to have already occurred.

This disclosure also provides therapeutic methods using *ex-vivo* or authologous transplantation techniques. Disclosed are *ex-vivo* therapeutic methods wherein the cytotoxic agent is administered to authologous cells before transplantation to patient. For example, a population of stem cells containing malignant cells and non-malignant cells were isolated from a patient. In the case of a cancer patient, normal stem cells and tumerogenic stem cells were isolated and then separated according to known methods. The cytotoxic agent (e.g., radiation) was applied to normal stem cells before transplanting back into the patient.

Cytotoxicity within the scope of the invention is a result of a broad range of elements and conditions including diseases, drugs and therapeutic regimens, environmental hazards, ionizing radiation, biological, chemical and radiological warfare, among others. The biological warfare agents include, by way of example and not limitation, *Bacillus anthracis* (anthrax), *Clostridium botulinum* toxin (botulism), *Yersinia pestis* (plague), *Variola major* (smallpox), *Francisella tularensis* (tularemia), Brucellosis, Cholera, *Clostridium perfringens* Toxin, Crimean-Congo Hemorrhagic Fever, Ebola Hemorrhagic Fever, Melioidosis, Q Fever, Ricin, Rift Valley Fever, Saxitoxin, *Staphylococcal enterotoxin* B, *Trichothecene mycotoxin,* and *Venezuelan Equine Encephalitis,* among others. Chemical warfare agents, including by way of example and not limitation, nerve agents, blood agents, blister agents (Lewisites, nitrogen mustards, sulfur mustards, and others) pulmonary agents, incapacitating agents (e.g., lachrymatory agents), and toxins, among others. Also encompassed within the scope of this invention is cytotoxicity caused by administration of a high dosage of a drug and/or adverse side-effect of a drug in a clinical setting.

Cytotoxic drugs are broadly defined herein to include wide variety of drugs including, by way of example and not limitation, chemotherapeutic drugs, radiotherapeutic drugs, adrenergics, antiasthmatics, antiarrhythmics, anticancer drugs, anti-AIDS medications, anti-parkinsonian drugs, Alzheimer's medications, somatomedins, cardiovascular and hypertensive drugs, diabetes medications, anti-viral agents, antisense peptides, anti-ulcer medications, sleep medications, PMS therapeutics, analgesics, endocrine/reproductive therapeutics, birth control medicaments, general hormone replacement therapeutics, pain medications, NSAIDs, epileptic medications, migraine headache medications, stroke medications, antibiotics, immunizations, addiction treatments, anxiolytics, anti-inflammatory, angiogenic or anti-angiogenic drugs, or a combination thereof among others.

The cellular toxicity within the scope of the invention includes a wide variety of toxicity including inflammation-associated tissue damage, loss of capability of modulation of cell proliferation, apoptosis, or other cellular damages and cell death.

The subjects exposed to cytotoxicity or being at risk of exposure to cytotoxicity are mammals, particularly humans. In one embodiment, the subjects are healthy individuals who have been subjected to cytotoxicity or who are at risk for incurring or exposure to cytotoxicity. In another embodiment, the subjects are asymptomatic or symptomatic patients exposed to or at risk of being exposed to drugs or radiation with cytotoxicity. In yet another embodiment, the subjects are military or private personnel who are exposed to or at risk of being exposed to cytotoxic agents and elements including ionizing radiation, biological warfare agents, chemical warfare agents or cytotoxicity caused by adverse environmental conditions, such as extreme temperature, cold, agitation, turbulence, etc.

Cytotoxic protective compounds are used to protect against cytotoxicity or ameliorate or treat one or more symptoms of cytotoxicity. In one embodiment the cytotoxic protective drug of the invention is an anti-cancer, anti-angiogenic, or an anti-mitotic drug or radiation protective agents. Radiation protective agents according to this invention reduce radiation toxicity without diminishing the therapeutic anti-tumor effect of radiation and without promoting radiation-induced carcinogenicity. The disclosed therapeutic methods protect normal cells and tissues from the effects of acute and chronic exposure to ionizing radiation.

Disclosed are therapeutic methods to protect individuals from deleterious effects of radiation induced cytotoxicity. The inventive method provides assays that require exposure of the *ex vivo* cells to the toxic element and extrapolate the results to determine efficacy of a radioprotectant drug *in vivo.* The optimum time of administration of the cytotoxic protective drug, before after and/or during exposure to the toxic element can be measured with utmost accuracy and precision without risking human lives. The inventive method allows for efficacy determination by calibrating the degree and timing of protection afforded by a radioprotectant to a human subject, measure survival and relate to dose and time of drug administration to demonstrate efficacy and deduce the optimum time window of protection.

The efficacy of the cytoprotective drug can be measured by analyzing biomarkers and survival parameters in the cytoprotective drug treated cells after irradiation to provide for a real human readout of efficacy of a cytotoxic protective drug without endangering humans. Molecular biological markers of radiation response could potentially be of use for monitoring the progress of radiation therapy, and even for predicting outcome early in a treatment regimen. Biomarkers of radiation exposure could also be an important tool for triage of individuals in potentially exposed populations after a radiologic accident or "dirty bomb" incident. Microarray hybridization analysis provides an attractive avenue for the discovery of potentially effective candidate radioprotective drugs. Once validated, a set of biomarkers could be developed into a simple rapid assay on a sensitive quantitative real-time multiplex platform Initial studies of *ex vivo* irradiated human peripheral white blood cells supported such an approach, documenting dose-response relationships with little variability among donors for a number of genes and confirming the feasibility of multiplex systems.

The cells respond to ionizing radiation by using complex signaling systems that recognize radiation damage to DNA and membrane lipids. When damage is found, it leads to the activation of various intracellular pathways that modulate the activity of genes controlling cellular responses such as apoptosis, cell cycle arrest or repair. Many of these pathways belong to the mitogen activated protein kinase (MAPK) superfamily. These pathways have been linked to the growth factor-mediated regulation of diverse cellular events such as proliferation, senescence, differentiation and apoptosis. Exposure of cells to ionizing radiation and a variety of other toxic stresses induce simultaneous compensatory activation of multiple MAPK pathways. These signals play a critical role in controlling cell survival and repopulation following irradiation in a cell-type dependent manner. Biomarkers in the cascade of protein kinases play a critical role in controlling cell survival and repopulation following exposure to ionizing radiation. Study of these biomarkers has revealed no significant difference between *in vivo* and *ex vivo* treatments with cytoprotective drugs.

The invention as disclosed herein provides for biological assay systems that can evaluate abnormal expression of biomarkers and parameters in a wide range of target cell populations that can be cultured and subjected to cytotoxic agents. The cell-based approach of the invention employs biological assays to measure biological parameters in target cells that mimic the general physiology or disease condition of the subject and correspond to fully-functioning cells in the body *in vivo.* In one embodiment, the cell-based assay of the invention is a cell proliferation assay. Conventional cell proliferation assays have measured either 3H-thymidine or 5-bromo-deoxyuridine (BrdU) incorporation. The BrdU assay can use microscopy, flow cytometry or absorbance. Colorimetric tetrazolium compounds, in particular 3 [4,5-dimethylthiazol-2ylj-2,5-diphenyltetrazolium bromide (MIT), (Mosmann, J. Immunol. Meth. 65, 666 (1983)), have also been used. Horowitz and King, J. Immunol. Meth. 244, 49-58 (2000)) developed a multi-well, murine colony-forming assay in soft agar whereby the enumeration of cell proliferation or inhibition was measured using the MIT calorimetric method. Results were equivalent to the colony forming assay. The number of target cells was reduced to 1.25 X10⁴ cells/ml, but only studied granulocyte/macrophage progenitor cells were tested and not stem cells, erythropoietic or megakaryopoietic progenitor cells.

In another embodiment, the cell-based assay of the invention comprises i*n vitro* colony forming assays. These assays have particular potential for detecting the growth potential of stem cell transplants in hematological malignancies. The use of stem cell transplants using peripheral blood, bone marrow and umbilical cord blood have recently increased dramatically. For *in vitro* colony forming assays, it is the proliferative potential of the cells being analyzed, and their ability to be stimulated by growth factors *in vitro* that are essential factors. This dependency on the amplification compartment inherent in the hematopoietic system is often overlooked, however without this component, colony-forming assays in general, and especially predictive hemotoxicity testing, would be difficult to perform. Under steady-state conditions, the proliferative status of primitive stem cells is considered to be quiescent, while the proportion of cells in cell cycle increases with stem cell maturity. Once the stem cell has become determined with respect to a cell lineage, it enters the amplification compartment for producing the large and constant number of mature cells. With entry into the cell cycle, however, the cell becomes vulnerable to exogenous agents including the cytotoxic drugs typically used in oncology. In the case of cytotoxic drug testing, the target cells have to be in cell cycle.

For any drug that relies on cell proliferation, the tissues most affected or damaged by toxicity are those actively engaged in cell proliferation, which includes the bone marrow and the gastrointestinal tract, among other tissues. It therefore follows that hemotoxicity testing could also usefully be extrapolated to, and predictive for, the effects of a potential drug on other proliferating tissues. Thus, the GM-CPC assay, for example, can be used to predict myelosuppression. The predictive quality of this assay has been proven by validation studies with alkylating agents. Additionally, however, if the maximum tolerated drug concentration for hematopoietic cells can be predicted, as provided by the present invention, hemotoxicity studies can become instrumental in drug discovery through its use a toxic/therapeutic index-based assay.

The widespread use of *in vitro* hematopoietic assays was initiated when soluble factors released by fibroblasts were shown to be capable of stimulating cells to form granulocyte-macrophage colonies in soft agar (Bradley & Metcalf, Aust. J. Exp. Biol. Med 44, 287-287 (1987); Pluznik & Sachs, Exp. Cell Res. 43, 553-553 (1966)). Colony forming assays (CFAs) for erythropoietic progenitor cells (McLeod et al., Blood 44, 617-534 (1974); Iscove et al., J. Cell Phyisol. 2-23 (1974); Axelrad et al., Haemopiesis in Culture 226-223 (1974)), along with other hematopoietic lineages that were developed. The use of cytotoxic drugs such as 5-fluorouracil--(Hodgson et al.; Exp. Hemat. 10, 26-36 (1982) and hydroxyurea (Rosendaal et al., Nature 264, 68-69 (1976) allowed the hierarchy within the stem cell compartment to be elucidated and *in vitro* assays for primitive stem cell populations to be developed.

In these classic assays, the more primitive the target cells, the longer it took to detect its progeny in the form of a colony. These assays rely on circulating populations of stem and progenitor cells in the peripheral blood that can be isolated and used for hematopoietic status monitoring and hemotoxicity testing. For example, granulocyte-macrophage colony-forming cell (GM-CFC) assay and the enumeration of CD34⁺ cells (stem and early progenitor cells) currently form the basis for hematopoietic stem cell transplantation. Colony-forming assays for leukemic cells are also available.

In one embodiment, the invention provides methods to determine the adverse side effects of drugs in human through combination of inventive *in vitro* and *ex-vivo* assays. The methods of the invention would benefit patients significantly because if cytotoxicity was detected early in time, the physician can rapidly detect the problem and offer an alternative therapy with reduced financial and health implications in hospitalization and medication costs and improved patient comfort and recovery. The drugs can be an approved drug, an experimental drug, or a potential new drug candidate.

While drug toxicity (especially hepatotoxicity) is the most frequent reason cited for withdrawal of an approved drug, no simple solution exists to adequately predict such adverse events. Simple cytotoxicity assays in HepG2 cells are relatively insensitive to human hepatotoxic drugs in a retrospective analysis of marketed pharmaceuticals. In one embodiment, a panel of pre-lethal mechanistic cellular assays is used to deliver a more sensitive approach to detect endpoint-specific drug toxicities. The panel of assays include, by way of example and not limitation, steatosis, cholestasis, phospholipidosis, reactive intermediates, mitochondria membrane function, oxidative stress, and drug interactions. Since inter-individual therapeutic index (TI) may differ from patient to patient, the rational use of one or more of these cellular assay and targeted *in vitro* exposure data according to the invention may allow pharmaceutical scientists to select drug candidates with a higher TI potential in the drug discovery phase without the need to expose human subjects to the deleterious and adverse side effects of these drugs.

For example, phospholipidosis, the accumulation of phospholipids in cells, is a relatively frequent side effect of cationic amphiphilic drugs. *In vitro* drug screening assays can be developed based on the prediction of the phospholipidosis-inducing potential of a drug candidate. High-throughput physicochemical approach can be used based on the measurement of drug-phospholipid complex formation observed by their effect on the critical micelle concentration (CMC) of a short-chain acidic phospholipid.

Comparison of results for hundreds of drugs to human data, animal testing, cell culture assays, and other screening methods reveals very good correlation to their phospholipidosis-inducing potential. The method is well suited for drug screening and therapeutic regimen to develop cytotoxic protective drugs that protects humans from the adverse side-effect of phospholipidosis. Such cytotoxic protective drugs may be administered in combination with the cationic amphiphilic drugs, or may itself be used as a cationic amphiphilic drug without the adverse side-effect.

The cationic amphiphilic drug, includes, by way of example and not limitation, antibacterial agents, antifungal agents, cytotoxic agents, antineoplastic agents, antiproliferative agents, immunosuppressants, disinfectants, antiseptic agents, antipsychotic agents and agents used in the treatment of cardiovascular diseases, among others. Some non limiting examples of a cationic amphiphilic drug are mitoxantrone, anthracycline-based drugs (*e.g*. daunorubicin, idarubicin, epinubicin, doxorubicin); camptothecin analogs (*e.g*. topotecan, irinotecan) inhibiting DNA Topoisomerase I enzyme; psychotropic drugs such as the desiprainine piperidine and piperazine-type neuroleptics, promazine, imipramine, amitriptyline, fluoxetine, sertraline; cardiovascular agents (*e.g*. venapamil or a calcium cannel blocker).

In yet another embodiment, the assay of the invention detects cytotoxicity developed by chemotherapy-induced peripheral neurotoxicity (CIPN). CIPN is a major clinical problem since it is the dose-limiting side effect of a significant number of antineoplastic drugs. The incidence of CIPN varies depending on the conditions, but severe neuropathy can occur in up to 40% of patients undergoing a polichemotherapy regimen. Moreover, even when CIPN is not a dose-limiting side effect, its onset may severely affect the quality of life of cancer patients and cause chronic discomfort. In recent years, new agents have been proposed as neuroprotectants, and some of them have been more specifically studied for CIPN. In the pre-clinical studies it has been shown that certain cytokines and growth factors have a protective role on selected neuronal targets. The assay system of the invention facilitates development of cytotoxic protective drugs against CIPN toxicity and determines the likelihood of development of CIPN in a given patient before initiation of chemotherapy.

The *in vitro, in vivo* and *ex vivo* assays according to the invention are conducted on target cells. Target cells refer to any cell population that may derive from normal or malignant tissues. In one embodiment, target cells are derived from hematopoietic stem and progenitor cells, or subpopulations thereof. The target cells of the invention are also utilized for target validation and the subsequent drug discovery. Target validation involves the experimental identification of a previously unknown function and/or functional association of a potential target with a physiological and/or disease or other pathophysiological phenotype. A target or validated target is a biomolecule whose function and/or functional association has been identified/verified in a validation process.

In one embodiment, the methods of the present invention are applied to populations of primitive hematopoietic cells including primary cells isolated from peripheral blood cells or bone marrow cells or to hematopoietic stem and progenitor cells. In another embodiment, the methods of the present invention are applied to cells isolated from tissues and solid tumors. However, the methods of the present invention may be applied to any population of proliferating or non-proliferating cells. The methods of the present invention may further be used to distinguish subpopulations of cells that may differ in response to cytotoxic agents.

In another embodiment, the target cells of the invention includes cell population having genetically-modified cells containing a nucleic acid molecule including a sequence of nucleotides encoding a target biomolecule, the target biomolecule having a confirmed functional association with a physiological and/or disease or other pathophysiological process.

The therapeutic regimen may be used to affect mobilizing, enhancing the trafficking of, and/or recruiting target stem cells and/or progenitor cells to the damaged tissue site, as well as the treatment of diseases associated with the inflammation that ensues from a variety of diseases and biological, chemical and radiological warfare.

The hematopoietic system is one of five continuously proliferating systems of the body, the others being the epithelial mucosa of the gastrointestinal tract, the dermis of the skin, the germ cells of the reproductive organs and the epithelium of the eye cornea. All five proliferating systems share common characteristics, the most important being that a small population of stem cells maintains the continuous production of mature end cells. They all possess the same structural organization of four basic compartments, namely the stem cell, amplification and differentiation, maturation and mature cell compartments.

The hematopoietic system, however, is unique in several ways. It is the only system capable of producing at least eight functionally different cell lineages from a single pluripotent stem cell. Second, the site of cell production changes during ontological development. Third, the site of production in the adult is the bone marrow, which is a significantly different tissue from the functional site of the peripheral circulation. Fourth, compared with other proliferating systems, and almost all other systems of the body, adult hematopoietic stem and progenitor cells are readily accessible.

Previously, hematopoietic stem cells were extracted by isolation from the bone marrow growth factor mobilized peripheral blood or cord blood (placenta). Recently, hematopoietic stem cells have been prepared from Embryonic Stem Cells, which are extracted from embryos obtained using *in vitro* fertilization techniques. These undifferentiated cells are capable of multi-lineage differentiation and reconstitution of all body tissue i. e. they are totipotent.

There are a number of undifferentiated stem cells of the hematopoietic lineage. These include pluripotent stem cells (PSCs), lymphoid stem cells (LSCs) and myeloid stem cells (MSCs), known collectively as lymphohaematopoietic progenitor cells (LPCs). LSCs and MSCs are each formed by the differentiation of PSCs. Hence, LSCs and MSCs are more committed than PSCs. Examples of differentiated cells of the hematopoietic lineage include T cells, B cells, eosinophils, basophils, neutrophils, megakaryocytes, monocytes, erythrocytes, granulocytes, mast cells, and lymphocytes. T cells and B cells are formed by the differentiation of LSCs. Hence, T cells and B cells are more committed than LSCs. In more detail, the chain of differentiation is LSC, pro-B-cell or prothymocyte, Pro-B-cell, pre-B-cell, mature B-cell, plasma cell, Prothymocyte common thymocyte, mature thymocytes (helper/inducer or cytotoxic/suppressor lineages). Eosinophils, basophils, neutrophils, megakaryocytes, monocytes, erythrocytes, granulocytes, mast cells, NKs, and lymphocytes are formed by the differentiation of MSCs. Hence, each of these cells are more committed than MSCs. In more detail, the chain of differentiation is MSC immature megakaryoblast, megakaryocyte, or proerythroblast (erythroblast, reticulocyte, erythrocyte) or myelomoriocytic stem cell, a bipotent stem cell that differentiates to either a myeloblast (promyelocyt, myelocyt, granulocyte) or a monoblast (promonocyte, monocyte, macrophage).

The pathways of differentiation of hematopoietic have thus been extensively characterized and the various cell stages are readily identifiable according to morphology and lineage-specific cell surface markers. In addition, the growth factors necessary to induce differentiation into various cell stages is also well-characterized.

The uniqueness of the hematopoietic system has important implications for toxicity screening of compounds. For example, the pluripotent nature of hematopoietic stem cells allows the differential effect and sensitivity of drugs on the various lympho-hematopoietic lineages to be examined. In addition, peripheral blood contains mature end cells that can be readily obtained to measure red and white blood cell counts, differential counts and other end stage blood parameters. These kinds of conventional parameters are typically used in preclinical drug testing and form the basis of the National Cancer Institute (NCI) guidelines for hemotoxicity testing during clinical trials.

Toxicity in general and hemotoxicity in particular, can be correlated with the time of drug administration. The therapeutic index of a drug, and hence its toxicity, is dependent, in part on the circadian variation in the hematopoietic cell division. Similarly, cells of the gut mucosa and the corneal epithelium of the eye exhibit circadian organization. For human bone marrow and gastrointestinal tissues, for example, S-phase DNA synthesis preferentially occurs in the morning hours rather than in the evening or nighttime hours. This implies cytotoxic agents might be less toxic and exhibit high efficacy if given at a time when the proliferative status of the cells is at a lowest point in these tissues.

The therapeutically effective amount or dosage of the cytotoxic protective drug of the present invention will depend on the disease state or condition being treated and other clinical factors such as weight and condition of the human or animal and the route of administration of the compound. The precise dose to be employed in the formulation, therefore, should be decided according to the judgment of the practitioner and each patient's circumstances. Effective doses may be extrapolated from dose-response curves derived from in vitro or animal model test systems.

For treating humans or animals, between approximately 0.5 to 500 mg/kilogram is typical broad range for administering the cytotoxic protective drug of the invention. For example, pretreatment with 120 mg/kg ON 01210.Na at 24 hours and 15 minutes before lethal doses of radiation provided evidence of survival benefit relative to vehicle-treated animals.

The disclosed methods contemplate single as well as multiple administrations, given either simultaneously or over an extended period of time. It is to be understood that the present invention has application for both human and veterinary use.

Preferred unit dosage formulations are those containing a daily dose or unit, daily sub-dose, as herein above recited, or an appropriate fraction thereof, of the administered ingredient. It should be understood that in addition to the ingredients, particularly mentioned above, the formulations of the cytotoxic protective drug may include other agents conventional in the art having regard to the type of formulation in question.

According to one aspect, the invention as disclosed herein is used in drug discovery and methodology to discover new cytotoxic protective drugs or discover new mechanism of action or new methods of using of a known marketed cytotoxic protective drug. Targets for drugs have so far been predicted on the basis of molecular or cellular features, for example, by exploiting similarity in chemical structure or in activity across cell lines. Methods to compare phenotypic side-effect similarities *in vitro* to infer whether two drugs share a target *in vivo* are also contemplated herein. The effectiveness of many therapies (including chemical, radiation, or surgery) today is severely limited by their harmful and hazardous side-effects. In order to maximize clinical value of a drug, effort must be taken to reduce or eliminate its side-effect on the normal tissues. The drug screening method of the invention is useful for finding candidate cytotoxic protective drugs that reduce or eliminate the side-effect of a primary drug on the normal cells, tissues and organs not affected by the underlying disease or disorder.

The combination of *in vitro* and *ex vivo* assays and targeting on therapeutically relevant or disease associated biological markers of the present invention provides for target validation and finally the resulting assays for drug discovery. Also encompassed within the scope of the invention is the functional identification of novel cytotoxic protective drug candidates as well as the functional characterization/validation of preexisting cytotoxic protective drugs in a cellular context that mimics the relevant general physiology or disease condition for a successful drug discovery effort.

In one embodiment, screening cytotoxic protective drugs are compounds by using a hematopoietic biomarker, or a binding fragment thereof. In another embodiment, the screening method is used for identifying candidate compounds including polypeptides, nucleotide and/or small molecules that bind biomarkers. Such a drug screening method would include, for example, contacting the candidate compounds of the present invention with a selected biomarker (s) suspected of being abnormally expressed in target cells exposed to toxicity and assaying the activity of these compounds following binding. These binding molecules or compounds are useful, for example, as agonists and antagonists of biomarkers. Such agonists and antagonists can be used, in accordance with the invention, in the therapeutic embodiments described in detail, below.

The compounds employed in such a test may be affixed to a solid support, expressed on a cell surface, free in solution, or located intracellularly. One method of drug screening utilizes eukaryotic or prokaryotic host cells which are stably transformed with recombinant nucleic acids expressing the biomarker or fragments thereof. Drugs are screened against such transformed cells in competitive binding assays. One may measure, for example, the formulation of complexes between the agent being tested and a biomarker according to this invention.

Another technique for drug screening provides high throughput screening for compounds having suitable binding affinity to one or more biomarkers. Briefly stated, large numbers of different small molecule or peptide test compounds are synthesized on a solid substrate, such as plastic pins or some other surfaces. The small molecule or peptide test compounds are reacted with the biomarkers that are expressed in target cells exposed to a cytotoxic agent. Bound small molecule and/or polypeptides are then detected by methods well known in the art.

This invention also contemplates the use of competitive drug screening assays in which neutralizing antibodies are used that are capable of binding biomarkers and specifically compete with a test compound for binding to a biomarker. In this manner, the antibodies are used to detect the presence of any peptide which shares one or more antigenic epitopes with a specific biomarker.

The plurality of candidate compounds may be provided by way of diversity libraries, such as random or combinatorial peptide or non-peptide libraries which can be screened for molecules that specifically bind to a biomarker. Many libraries are known in the art that can be used, i.e., chemically synthesized libraries, recombinant (i.e., phage display libraries), and *in vitro* translation-based libraries. Examples of chemically synthesized libraries are described in Fodor et al., Science 251:767-773 (1991); Houghten et al., Nature 354:84-86 (1991); and Brenner and Lemer, Proc. Natl. Acad. Sci. USA 89:5381-5383 (1992), among others. By way of examples of non-peptide libraries, a benzodiazepine library (*see, i.e.,* Bunin et al., Proc. Natl. Acad. Sci. USA 91:4708-4712 (1994)) can be adapted for use. Peptoid libraries (Simon et al., Proc. Natl. Acad. Sci. USA 89:9367-9371 (1992)) can also be used. Another example of a library that can be used, in which the amide functionalities in peptides have been permethylated to generate a chemically transformed combinatorial library, is described by Ostresh et al., Proc. Natl. Acad. Sci. USA 91:11138-11142 (1994).

The non-peptide libraries that are useful in the present invention are, for example, libraries described by Ecker and Crooke, Bio/Technology 13:351-360 (1995). These libraries use compounds such as, for example, benzodiazepines, hydantoins, piperazinediones, biphenyls, sugar analogs, beta-mercaptoketones, arylacetic acids, acylpiperidines, benzopyrans, cubanes, xanthines, aminimides, and oxazolones among others to form the basis of various libraries. Non-peptide libraries can be classified broadly into two types: decorated monomers and oligomers. Decorated monomer libraries employ a relatively simple scaffold structure upon which variety of functional groups added. Often the scaffold will be a molecule with a known useful pharmacological activity. For example, the scaffold might be α, β unsaturated aryl sulfone benzodiazepine.

The present invention also provides the use of test kits in the claimed methods, for example to screen for candidate cytotoxic protective drug or determine efficacy of a known cytoprotective drug in a clinical setting. The test kits includes solutions and devices for extraction of target cells from the subject, one or more test compounds as potential cytotoxic protective drugs, cell and tissue culture media and plates, one or more cytotoxic agents, biomarkers and assays to analyze parameters indicative of viability and growth conditions in target cells, one or more vessels containing the necessary reagent mixes and instructions for the use thereof for performing the high-throughput assays for detecting and measuring the cytotoxic protective potential of a candidate drug. The test kits of the invention may be used to perform high-throughput assays to determine the cytotoxic preventive potential of a candidate compound.

In another embodiment, the use of the test kits may be for screening and determining efficacy and pharmcodynamics of a therapeutic regimen to protect a subject from deleterious effects of a cytotoxic agent comprising reagent mixes and instructions for performing high-throughput assays, solutions and devices for extracting target cells from the subject, a vial containing the cytotoxic agent, a vial containing the cytotoxic protective compound, cell and tissue culture media and plates, assay components for determination of viability and growth of target cells *in vitro,* and instructions for the use of different components of the kit.

### EXAMPLES

The invention now being generally described, it will be more readily understood by reference to the following examples, which are included merely for purposes of illustration of certain aspects and embodiments of the present invention, and are not intended to limit the invention, as one skilled in the art would recognize from the teachings hereinabove and the following examples, that other DNA microarrays, neurological conditions, cognitive therapies or data analysis methods, all without limitation, can be employed, without departing from the scope of the invention as claimed.

### Example 1 Primary Pharmacodynamics: Dose-Dependent Radioprotection by ON 01210.Na in Normal Human Fibroblasts In Vitro

A clonogenic assay was used to measure radioprotection of ON 01210.Na on cultured human cells. Treatment of HFL-1 (normal diploid lung fibroblasts) cells with 2-20 µM of ON 01210.Na for 24 hours prior to cytotoxic levels of Ionizing Radiation (IR) (10 Gy) provided 3.3-4.7 fold protection in a dose-dependent manner (see Figure 1A).HFL-1 cells, were treated with ON 01210.Na at final concentrations ranging from 2.0 to 20.0 µM. In a second experiment, cells were also treated with the drug for 24 h, but the drug was applied 4 h after irradiation.

Figure 1 demonstrates protection of HFL-1 cells against IR by ON 01210.Na. Following drug treatment and radiation exposure, cells were re-plated and the number of colonies from each plate was enumerated three weeks later. A. Relation of concentration of ON 01210.Na to number of surviving colonies. B. Stained clonogenic assay plates.

An important observation, relevant to field-use in post-radiation exposure scenarios, was that significant protection was afforded when the cells were treated with ON 01210.Na four hours post irradiation exposure. In addition to the increase in total number of surviving colonies afforded by ON 01210.Na treatment, consistently, the number of cells per colony was also significant larger in the ON 01210.Na treated group (see Figure 2B). This suggested that the post-IR repair mechanisms in the ON 01210.Na treated cells increased not only the number of surviving cells but also the proliferative capabilities of the protected cells.

### Example 2 In Vitro Radiation Protection of Human Bone Marrow Cells

The ability of ON 01210.Na to protect primary human bone marrow cells from cytotoxic doses of radiation was studied in an *in vitro* assay. Initially, cytotoxicity studies were performed. Human bone marrow cells isolated from healthy volunteers were treated with increasing concentrations of ON 01210.Na for 2 and 24 hours and plated into methocult. These studies clearly showed that ON 01210.Na treatment was non cytotoxic even at high concentrations (20 µM) over a 24 hour treatment period (Figure 2).

Radiation protection assays were then performed (Figure 3). Cells were treated with ON 01210.Na for 3 hours, irradiated, and plated in methocult. The total number of colony forming units was determined 14 days later. ON 01210.Na was found to protect human bone marrow cells in a dose dependent manner, with an optimal DRF of 1.6. This compared very favorably when analyzed against protection studies using amifostine as a positive control. These studies showed that ON 01210.Na was non-toxic to human bone marrow cells and was radioprotective activity for primary human bone marrow cells. Figure 2. ON 01210.Na was not cytotoxic to human bone marrow cells *in vitro.* Human bone marrow cells were treated with various concentrations of ON 01210.Na for (A) 2 hrs or (B) 24 hrs. The cells were then plated into methocult medium. Total colony forming units were determined 14 days later. ON 01210.a did not interfere with the growth or differentiation of human bone marrow cells even after long-term exposure at high concentrations.

Figure 3 shows i*n vitro* radiation protection of human bone marrow cells. Human bone marrow cells were treated with increasing concentrations of ON 01210.Na for 3 hours, and then irradiated with increasing doses of ionizing radiation. The cells were plated into methocult and the total number of CFUs was determined 14 days later. ON 01210.Na treatment without radiation was non-toxic at all concentrations tested (A). ON 01210.Na protected human bone marrow cells in a dose responsive manner (B). When the survival curves were plotted at the optimal ON 01210.Na concentration (10 uM), ON 01210.Na (C) had a DRF value of 1.6 while the optimal dose of amifostine (0.25 mM) had a DRF value of 1.5.

As demonstrated in Figure 3, ON 01210.Na is very effective at protecting human bone marrow cells from radiation exposure. The 3 hour ON 01210.Na treatment was completely safe (Figure 3A) to the human bone marrow cells. ON 01.210.Na radiation protection was found to be dose-dependent (Figure 3B). The protection data indicated that a concentration of ON 01210.Na above 10 µM for 3 hours did not increase the amount of radiation protection suggesting that under these assay conditions 10 µM was the optimal concentration for radiation protection. When we plotted the survival curve at the optimal concentration of ON 01210.Na (Figure 3C) we were able to calculate a dose reduction factor (DRF) of 1.6. We also tested the radiation protection activity of amifostine. Amifostine concentrations were selected that closely mimicked the therapeutic plasma concentrations. ON 01210.Na protected cells in a broad range of concentrations (10 fold) but amifostine only protected human bone marrow cells in a narrow range of concentrations (data not shown), with the optimal being 0.25 mM. This concentration was found to be optimal in previous studies using mesenchymal and hematopoietic cellsii. Figure 3D shows the survival curve when human bone marrow cells were treated with amifostine. The calculated DRF value of 1.5 was determined from these experiments. This data clearly indicates that ON 01210.Na was as effective at protecting human bone marrow cells from radiation exposure as amifostine, but at a wider range of safe concentrations.

### Example 3 ON 01210.Na Protection of Murine Hematopoietic Cells (In Vivo Radiation Protection Assay)

Since survival of animals after radiation exposure depends largely on the protection of the hematopoietic system, the effect of ON 01210.Na treatment on hematopoietic cell survival was investigated using various schedules before radiation exposure. The radioprotection of hematopoietic cells by ON 01210.Na was studied in an *in vivo* assay using a sub lethal dose of radiation. C3H/HEJ mice were treated with ON 01210.Na following various schedules in order to determine the optimal dose and schedule of ON 01210.Na for maximum protection of the hematopoietic system in mice. Previous survival studies showed that a double injection of ON 01210.Na, minus 24 hour and minus 15 minutes, as well as a single injection at minus 15 minutes prior to lethal exposure of ionizing radiation protected 100% of the mice. The following hematopoietic studies showed that the survival studies correlated closely with protection of the hematopoietic cells. The double injection, minus 24 hour and minus 15 minutes, and the single, minus 15 minutes, injections of ON 01210.Na resulted in significant recovery of both the colony forming units and the white blood cells of irradiated mice. A single injection 4 hours prior to radiation exposure protected the mice from complete ablation of the bone marrow compartment, but did not show a significant advantage in recovery time over vehicle alone.

ON 01210.Na had a significant protective effect on the recovery of both the bone marrow compartment and the peripheral blood compartment following radiation exposure. The results also showed that schedule of ON 01210.Na treatment that resulted in 100% survival correlated very closely with the recovery of the hematopoietic system. Figure 4 demonstrated that ON 01210.Na protects the bone marrow of irradiated mice. A. Mice were injected with 500 mg/kg of ON 01210.Na formulated in 1% Polysorbate 80/K-phosphate buffer or vehicle alone by sc injections minus 24 hrs and minus 15 minutes before 5.5 Gy of whole body ionizing radiation treatment. Whole blood was collected on the indicted days and WBC counts were performed following RBC lysis. The data is plotted as the average of three mice per group +/-SEM (N:3x3=9 plates). B. Pictures of representative plates. ON 01210.Na protected the bone marrow from complete ablation of stem cells and significantly increased the recovery of the bone marrow. C. White blood cell counts. Whole blood was collected on the indicted days and WBC counts were performed following RBC lysis. The data is plotted as the average of 3 mice per group ±SEM (N=3). Lineage specific protection was studied by identifying and tabulating three lineages, granulocytes, macrophages and granulocyte-macrophages (GM). Table 4 shows the number of cells of each lineage following radiation exposure.

**Table 1. Differential Colony Counts.**

| **Group** | **Cell Type** | **Day** | | | |
|---|---|---|---|---|---|
| | | **4** | **7** | **12** | **18** |
| **Vehicle No IR** | Total CFU | 149 | 119 | 196 | 154 |
| | Macrophages | 7 | 5 | 10 | 9 |
| | Granulocytes | 28 | 18 | 69 | 48 |
| | GM | 114 | 96 | 117 | 96 |
| **ON 01210.Na No IR** | Total CFU | 147 | 143 | 194 | 197 |
| | Macrophages | 9 | 5 | 14 | 39 |
| | Granulocytes | 27 | 18 | 64 | 39 |
| | GM | 111 | 120 | 116 | 119 |
| **Vehicle** | Total CFU | 1.89 | 8 | 12 | 48 |
| **5.5 Gy IR** | Macrophages | 0.56 | 0.8 | 0.4 | 8 |
| | Granulocytes | 0.78 | 4 | 5 | 21 |
| | GM | 0.56 | 3 | 7 | 18 |
| **ON 01210.Na 5.5 Gy IR** | Total CFU | 21 | 24 | 37 | 146 |
| | Macrophages | 4 | 2 | 5 | 16 |
| | Granulocytes | 9 | 13 | 16 | 58 |
| | GM | 8 | 9 | 16 | 72 |

Recovery was observed in all three lineages with a slight bias towards the macrophage lineage. There was a slight increase in the macrophage colony number when the mice were injected with ON 01210.Na without radiation exposure. These results show that, not only was ON 01210.Na able to significantly increase the rate of recovery of bone marrow progenitor cells, but it was also able to protect an existing fraction of cells. In addition to examining the ability of ON 01210.Na to protect bone marrow cells, the protection of peripheral white blood cells was also examined. Whole blood was collected and the total number of white blood cells was determined at various times after radiation exposure. Figure 4C shows that ON 01210.Na significantly protected white blood cells from radiation exposure. Mice treated with ON 01210.Na also displayed a more rapid recovery of white cell numbers by 18 days.

In order to determine the optimal schedule of ON 01210.Na treatment for maximum protection of the bone marrow, bone marrow protection of mice injected with a single dose of ON 01210.Na at minus 15 minutes or minus 4 hours was compared with mice injected with two doses described above. The results from these experiments are shown in Table 5. The most efficient protection of the bone marrow was found when the mice were injected at minus 24 hours and minus 15 minutes before radiation exposure. A single injection just before exposure was very effective at protecting bone marrow cells and inducing recovery of the bone marrow cells following radiation exposure. If ON 01210.a was injected as a single dose at 4 hours before radiation exposure, protection of bone marrow cells was observed but the recovery rate was not significantly increased as compared to vehicle treated mice. These experiments imply that a single injection just before exposure will protect and increase the recovery of bone marrow cells after radiation exposure.

**Table 2. Per Cent Colony Forming Units after Different Schedules of Treatment.**

| | | **Time of Administration Relative to IR Exposure** | | |
|---|---|---|---|---|
| **Day** | **Vehicle** | **- 24 h and -15 min** | **-15 min** | **-4 h** |
| **4** | 0 | 14 | 9 | 6 |
| **7** | 1 | 16 | 13 | 10 |
| **12** | 8 | 25 | 17 | 12 |
| **18** | 50 | 100 | 80 | 51 |

ON 01210.Na treatment of mice by subcutaneous injection significantly protected the bone marrow and white blood cells of irradiated mice. In addition to the protection of the hematopoietic cells, there was a dramatic increase in the rate of recovery of the bone marrow compartment. The optimal dose (500 mg/kg) and schedules (two injections minus 24 hour and minus 15 minutes or single minus 15 minutes prior to radiation treatment) for protection of the hematopoietic cells correlated with the dose and schedule found for optimal survival following exposure to lethal doses of radiation. Therefore, one important mechanism of radiation protection by ON 01210.Na is protection of the hematopoietic system.

### Example 4. Pharmacological Studies

Studies were conducted to determine the efficacy of cytotoxic protective drugs. In one study, cytotoxic/radioprotective drug ON 1210.Na, otherwise known as EX-RAD™ was investigated using both *in vitro* and *in vivo* model systems. ***In** vitro* studies used several established cell lines and freshly isolated human bone marrow cells. *In vivo* efficacy studies focused on the effects of ON 01210.Na treatment on post-radiation survival and the ability to enhance recovery of hematological and gastrointestinal systems. Two animal species were tested, mice and rabbits. In addition to efficacy work, several experiments were designed to explore potential mechanisms of protection at subcellular and molecular levels.

Efficacy studies demonstrated that cell-based experiment showed that ON 01210.Na protected human lung and skin fibroblasts and human umbilical vein endothelial cells from radiation exposure, using *in vitro* clonogenic survival assays. Furthermore, ON 01210.Na was nontoxic and able to reproducibly protect freshly isolated human donor bone marrow cells from high doses of radiation. The protective activity of ON 01210.Na was equivalent to amifostine treatment of the same isolates of human bone marrow cells.

*In vivo* survival studies in mice were designed to explore the effects of dose and schedule on survival, based on the well-established 30-day survival model, establish a dose reduction factor (DRF) for ON 01210.Na, compare two different formulations (suspension vs. solution) of ON 01210.Na; and show protection and recovery benefits of ON 01210.Na to the hematopoietic system and gastrointestinal tract of treated mice.

In mice, ON 01210.Na demonstrated a dose-dependent protection against radiation lethality at doses ranging from 50 mg/kg to 500 mg/kg (p < 0.05 vs.vehicle). At 500 mg/kg, survival studies using the LD50/30 whole body radiation dose showed that ON 01210.Na pretreatment completely and reproducibly protected mice from exposure. Dose-response studies showed that a 500 mg/kg dose, given subcutaneously as a suspension, at 24 hours and 15 minutes prior to radiation exposure, was an optimal dose and schedule of ON 01210.Na treatment. Two dose reduction factor (DRF) studies determined that the DRF for ON 01210.Na was 1.14 to 1.16. This dosing regimen using a solution formulation was shown to be similarly effective in mice.

Analysis of the hematopoietic system of mice treated with ON 01210.Na prior to sublethal doses of ionizing radiation showed that both the progenitor bone marrow stem cells and the peripheral blood cells were protected against acute radiation exposure by ON 01210.Na treatment. In addition, the gastrointestinal (GI) tissue of mice pretreated with ON 01210.Na prior to irradiation showed significantly less damage to the jejunal crypt stem cells.

In three survival studies with New Zealand white rabbits, pretreatment with 120 mg/kg ON 01210.Na at 24 hours and 15 minutes before lethal doses of radiation provided evidence of survival benefit relative to vehicle-treated animals. There was a general protective trend observed for white blood cells, platelets, hematocrit and hemoglobin levels. Unlike in the mouse studies, clear evidence of improved repopulation of various hematologic cell lineages was not evident in these preliminary studies. Both solution and suspension formulations were tested.

These efficacy studies taken together showed that ON 01210.Na has significant *in vitro* radiation protection activity across a number of cell lines, and in primary human bone marrow cells *in vitro*. ON 01210.Na afforded survival benefit to mice exposed to lethal doses of whole body ionizing radiation in a dose dependent and time of treatment dependent manner. ON 01210.Na was shown to promote survival of, and enhance recovery of peripheral hematopoietic and progenitor crypt stem cells in mice exposed to whole body radiation. In the large animal model, we showed that ON 01210.Na was able to protect rabbits from lethal doses of whole body radiation when given prior to radiation exposure. Additionally, ON 01210.Na did not exhibit any significant activity as a free radical scavenger or antioxidant; ON 01210.Na treatment was not cytotoxic and did not cause cell cycle arrest; and ON 01210.Na treatment reduced the amount of DNA damage caused by irradiation of cells, as assessed by "Comet" assays.

### Example 5. Development of Biological Assays

To analyze and investigate the efficacy of a cytotoxic protective drug, biological assays were developed that assisted in determination of the mechanism of action of these drugs at the intracellular level. In the case of ON 01210.Na, certain components of cellular and DNA damage-sensing, repair, and apoptosis pathways were studied in more detail. It was found that ON 01210.Na treatment prior to radiation exposure did not adversely affect the cells' early response to IR exposure, but influenced later transducer signaling events that contribute to reduction in cellular apoptosis caused by the signaling cascade activated upon exposure to radiation. The signaling studies suggested that, in addition to the decrease of DNA damage by 24 hours after exposure to radiation, ON 01210.Na treatment also reduced the level of apoptosis-specific signaling events, and hence led to increased recovery of the cells instead of cell death.

In addition, ON 01210.Na was found to significantly reduce the protein levels of p53, p21, bax, c-abl and p73 in cells in response to radiation exposure. This reduction could be instrumental in decreased cell death and increased cell recovery in ON 01210.Na pretreated irradiated cells.

Additional biochemical studies were performed to screen for potential secondary or non-specific activities. ON 01210.Na was subjected to *in vitro* primary screening across a broad panel of 67 radioligand receptor binding assays and five cytochrome P450 enzymes: 1A2, 2C9, 2C19, 3A4, and 2D6. No significant inhibition or stimulation of receptor binding was detected. Only one cytochrome assay, CYP450 2C9, in which the activity was stimulated 98%, yielded a response that was considered significant. Thus, as a preliminary screen for safety and selectivity profiling, no targets were identified.

## Claims

1. A method of determining efficacy of a therapeutic regimen to protect a subject from deleterious effects of a cytotoxic agent comprising:
i) exposing a) target cells extracted from a subject prior to treatment with a cytotoxic protective drug, and b) target cells extracted from the subject after treatment with the cytotoxic protective drug, to the cytotoxic agent; and
ii) analyzing and comparing one or more parameters indicative of viability and growth conditions in target cells of step i),
wherein a favorable viability and growth condition of the target cells of step ib) as compared to target cells of step ia) is indicative of the efficacy of the therapeutic regimen in the subject.

2. The method of Claim 1, further comprising addition of the cytotoxic protective drug *in vitro* to the target cells of step ia) before exposure to the cytotoxic agent.

3. The method of Claim 1, wherein the target cells comprise cells that are specifically affected by the cytotoxic agent upon exposure.

4. The method of Claim 2, wherein the target cells comprise epithelial tissues, connective tissues (blood, bone, cartilage), muscle tissues, nerve tissues, stem tissues and cells, progenitor cells, and subclasses and categories of these tissues and cells.

5. The method of Claim 4, wherein the target cells comprise stem cells from hematopoietic system, epithelial mucosa of the gastrointestinal tract, the dermis of the skin, the germ cells of the reproductive organs, the epithelium of the eye cornea, or a combination thereof.

6. The method of Claim 5, wherein the stem cell from hematopoietic system comprises stem cell and progenitor cells from bone marrow.

7. The method of Claim 1, wherein the cytotoxic agent comprises a chemical cytotoxic agent, a radiological cytotoxic agent, an ionizing radiation, a biological cytotoxic agent, a biological warfare agent, a chemical warfare agent, a drug with adverse side effects in humans, or a combination thereof.

8. The method of Claim 1, wherein the cytotoxic protective drug comprises small molecule compounds, peptides, gene expression products, chemoprotective drugs, radioprotective drugs, antiviral or antibacterial drugs, angiogenic or anti-angiogenic drugs, anti-inflammatory drugs.

9. The method of Claim 8, wherein the radioprotective drug exhibits a synergistic anti-cell proliferation activity in tumor cells of cancer patients when administered with chemotherapeutic agents or radiation.

10. The method of Claim 8, wherein the radioprotective drug is a non-toxic radioprotective drug comprising compounds of α, β unsaturated aryl sulfones.

11. The method of Claim 10, wherein the compound of α, β unsaturated aryl sulfones comprises E-4-Carboxystyryl-4-chlorobenzylsulfone sodium salt.

12. The method of Claim 8, wherein the cytotoxic protective drug exerts radioprotection by inhibiting the activities of radiation-sensitive genes, inhibition of apoptosis, and protecting DNA from radiation- induced damage, or a combination thereof

13. The method of Claim 1, wherein the viability and growth condition of the target cells are determined by analyzing one or more parameters or biomarkers that are up- regulated, down-regulated, or abnormally expressed in the target cells upon exposure to the cytotoxic agent.

14. The method of Claim 13, wherein the one or more parameters or biomarkers are nucleic acid or peptide molecules related to intracellular pathways modulating activity of genes controlling one or more cellular responses, gene expression products, biochemical pathways, protein cascades, cell receptors, pre-apoptosis signaling pathways and molecules, antigen or antibody fragments, agonists or antagonists of peptides, cytogenetics markers, or a combination thereof.

15. The method of Claim 14, wherein the one or more parameters comprise hematopoietic regulatory messenger biomarkers.

16. The method of Claim 14, wherein the intracellular pathways are involved in apoptosis, cell cycle arrest or repair, proliferation, senescence, or differentiation of target cells.

17. The method of Claim 14, wherein the intracellular pathways belong to mitogen activated protein kinase superfamily.

18. A method of screening for cytotoxic protective drug comprising:
i) exposing a) target cells extracted from a non-human subject prior to treatment with a cytotoxic protective test compound, and b) target cells extracted from the non-human subject after treatment with the cytotoxic protective test compound, to the cytotoxic agent; and
ii) analyzing and comparing one or more parameters indicative of viability and growth conditions in target cells of step i),
wherein a favorable viability and growth condition of the target cells of step ib) as compared to target cells of step ia) is indicative of the potential of the cytotoxic protective test compound for development as cytotoxic protective drug.

19. Use of a test kit in the method of claim 1 or claim 18, wherein the test kit comprises solutions and devices for extraction of target cells from the subject, one or more test compounds as potential cytotoxic protective drugs, cell and tissue culture media and plates, one or more cytotoxic agents, biomarkers and assays to analyze parameters indicative of viability and growth conditions in target cells, one or more vessels containing the necessary reagent mixes and instructions for the use thereof for performing the assay.

## Patentansprüche

1. Verfahren zum Bestimmen der Wirksamkeit eines Therapieprogramms zum Schützen eines Subjekts vor schädlichen Wirkungen eines zytotoxischen Mittels, umfassend:
i) Exponieren von a) Zielzellen, die vor der Behandlung mit einem Zytotoxikum-schützenden Arzneimittel aus einem Subjekt extrahiert wurden, und b) Zielzellen, die nach der Behandlung mit dem Zytotoxikum-schützenden Arzneimittel aus dem Subjekt extrahiert wurden, gegenüber dem zytotoxischen Mittel; und
ii) Analysieren und Vergleichen eines oder mehrerer Parameter, die für die Lebensfähigkeit und den Wachstumszustand indikativ sind, in Zielzellen aus Schritt i),
wobei günstige Lebensfähigkeit und günstiger Wachstumszustand der Zielzellen aus Schritt ib) im Vergleich zu Zielzellen aus Schritt ia) für die Wirksamkeit des Therapieprogramms in dem Subjekt indikativ sind.

2. Verfahren gemäß Anspruch 1, ferner umfassend Zugeben des Zytotoxikum-schützenden Arzneimittels *in vitro* zu den Zielzellen aus Schritt ia) vor der Exposition gegenüber dem zytotoxischen Mittel.

3. Verfahren gemäß Anspruch 1, wobei die Zielzellen Zellen umfassen, die bei Exposition von dem zytotoxischen Mittel spezifisch geschädigt werden.

4. Verfahren gemäß Anspruch 2, wobei die Zielzellen Epithelgewebe, Bindegewebe (Blut, Knochen, Knorpel), Muskelgewebe, Nervengewebe, Stammgewebe und -zellen, Progenitorzellen und Unterklassen und -kategorien dieser Gewebe und Zellen umfassen.

5. Verfahren gemäß Anspruch 4, wobei die Zielzellen Stammzellen aus dem hämatopoetischen System, Epithelmukosa des Gastrointestinaltrakts, die Dermis der Haut, die Keimzellen der Reproduktionsorgane, das Epithel der Kornea des Auges oder eine Kombination davon umfassen.

6. Verfahren gemäß Anspruch 5, wobei die Stammzellen aus dem hämatopoetischen System Stammzellen und Progenitorzellen aus Knochenmark umfassen.

7. Verfahren gemäß Anspruch 1, wobei das zytotoxische Mittel ein chemisches zytotoxisches Mittel, ein radiologisches zytotoxisches Mittel, eine ionisierende Strahlung, ein biologisches zytotoxisches Mittel, einen biologischen Kampfstoff, einen chemischen Kampfstoff, ein Arzneimittel mit schädlichen Nebenwirkungen in Menschen oder eine Kombination davon umfasst.

8. Verfahren gemäß Anspruch 1, wobei das Zytotoxikum-schützende Arzneimittel kleinmolekulare Verbindungen, Peptide, Genexpressionsprodukte, chemoprotektive Arzneimittel, radioprotektive Arzneimittel, antivirale oder antibakterielle Arzneimittel, angiogene oder antiangiogene Arzneimittel, entzündungshemmende Arzneimittel umfasst.

9. Verfahren gemäß Anspruch 8, wobei das radioprotektive Arzneimittel eine synergistische Antizellproliferations-Aktivität in Tumorzellen von Krebspatienten zeigt, wenn es mit chemotherapeutischen Mitteln oder Strahlung verabreicht wird.

10. Verfahren gemäß Anspruch 8, wobei das radioprotektive Arzneimittel ein nichttoxisches radioprotektives Arzneimittel ist, das Verbindungen von α,β-ungesättigten Arylsulfonen umfasst.

11. Verfahren gemäß Anspruch 10, wobei die Verbindung von α,β-ungesättigten Arylsulfonen E-4-Carboxystyryl-4-chlorbenzylsulfon-Natriumsalz umfasst.

12. Verfahren gemäß Anspruch 8, wobei das Zytotoxikum-schützende Arzneimittel Radioprotektion durch Hemmen der Aktivität von strahlungsempfindlichen Genen, Hemmen von Apoptose und Schützen von DNA vor strahlungsinduzierter Schädigung oder eine Kombination davon ausübt.

13. Verfahren gemäß Anspruch 1, wobei die Lebensfähigkeit von und der Wachstumszustand der Zielzellen durch Analysieren eines oder mehrerer Parameter oder Biomarker durchgeführt wird, die nach Exposition gegenüber dem zytotoxischen Mittel hochreguliert, herabreguliert oder in den Zielzellen anomal exprimiert werden.

14. Verfahren gemäß Anspruch 13, wobei der eine oder die mehreren Parameter oder Biomarker Nukleinsäure- oder Peptidmoleküle sind, die mit intrazellulären Signalwegen in Verbindung stehen, die die Aktivität von Genen modulieren, die eine oder mehrere zelluläre Antworten, Genexpressionsprodukte, biochemische Signalwege, Proteinkaskaden, Zellrezeptoren, Präapoptose-Signalwege und -moleküle, Antigen- oder Antikörperfragmente, Agonisten oder Antagonisten von Peptiden, Zytogenmarker oder eine Kombination davon steuern.

15. Verfahren gemäß Anspruch 14, wobei der eine oder die mehreren Parameter hämatopoetischregulatorische Messenger-Biomarker umfassen.

16. Verfahren gemäß Anspruch 14, wobei die intrazellulären Signalwege bei der Apoptose, dem Anhalten oder Wiederherstellen des Zellzyklus, der Proliferation, Seneszenz oder Differenzierung von Zielzellen beteiligt sind.

17. Verfahren gemäß Anspruch 14, wobei die intrazellulären Signalwege zu der mitogenaktivierte-Proteinkinase-Superfamilie gehören.

18. Verfahren zum Screening auf Zytotoxikum-schützende Arzneimittel, umfassend:
i) Exponieren von a) Zielzellen, die vor der Behandlung mit einer Zytotoxikum-schützenden Prüfverbindung aus einem nicht-menschlichen Subjekt extrahiert wurden, und b) Zielzellen, die nach der Behandlung mit der Zytotoxikum-schützenden Prüfverbindung aus dem nicht-menschlichen Subjekt extrahiert wurden, gegenüber dem zytotoxischen Mittel; und
ii) Analysieren und Vergleichen eines oder mehrerer Parameter, die für die Lebensfähigkeit und den Wachstumszustand indikativ sind, in Zielzellen aus Schritt i),
wobei günstige Lebensfähigkeit und günstiger Wachstumszustand der Zielzellen aus Schritt ib) im Vergleich zu Zielzellen aus Schritt ia) für das Potenzial der Zytotoxikum-schützenden Prüfverbindung für die Entwicklung als Zytotoxikum-schützendes Arzneimittel indikativ sind.

19. Verwendung eines Testkits bei dem Verfahren gemäß Anspruch 1 oder Anspruch 18, wobei das Testkit Lösungen und Vorrichtungen zum Extrahieren von Zielzellen aus dem Subjekt, eine oder mehrere Prüfverbindungen als potenzielle Zytotoxikum-schützende Arzneimittel, Zell- und Gewebekulturmedien und -Platten, ein oder mehrere zytotoxische Mittel, Biomarker und Assays zum Analysieren von Parametern, die für Lebensfähigkeit und Wachstumszustand von Zielzellen indikativ sind, einen oder mehrere Behälter, die die erforderlichen Reagenzmittelgemische enthalten, und Anleitungen für die Verwendung davon zum Durchführen des Assays umfasst.

## Revendications

1. Procédé de détermination de l'efficacité d'un régime thérapeutique pour protéger un sujet contre les effets délétères d'un agent cytotoxique, comprenant les étapes suivantes:
i) exposer a) des cellules cibles extraites chez un sujet avant un traitement avec un médicament de protection contre la cytotoxicité, et b) des cellules cibles extraites chez le sujet après un traitement avec le médicament de protection contre la cytotoxicité, à l'agent cytotoxique; et
ii) analyser et comparer un ou plusieurs paramètre(s) indicatif(s) de conditions de viabilité et de croissance dans les cellules cibles de l'étape i),
dans lequel une condition favorable de viabilité et de croissance des cellules cibles de l'étape ib) comparativement aux cellules cibles de l'étape ia) est indicative de l'efficacité du régime thérapeutique chez le sujet.

2. Procédé selon la revendication 1, comprenant en outre l'addition du médicament de protection contre la cytotoxicité *in vitro* aux cellules cibles de l'étape ia) avant l'exposition à l'agent cytotoxique.

3. Procédé selon la revendication 1, dans lequel les cellules cibles comprennent des cellules qui sont spécifiquement affectées par l'agent cytotoxique lors de l'exposition.

4. Procédé selon la revendication 2, dans lequel les cellules cibles comprennent des tissus épithéliaux, des tissus conjonctifs (sang, os, cartilage), des tissus musculaires, des tissus nerveux, des tissus et des cellules souches, des cellules progénitrices ainsi que des sous-classes et catégories de ces tissus et cellules.

5. Procédé selon la revendication 4, dans lequel les cellules cibles comprennent des cellules souches issues du système hématopoïétique, de la muqueuse épithéliale du tractus gastro-intestinal, du derme de la peau, les cellules souches des organes reproducteurs, l'épithélium de la cornée oculaire ou une combinaison de ceux-ci.

6. Procédé selon la revendication 5, dans lequel la cellule souche issue du système hématopoïétique comprend une cellule souche et des cellules progénitrices issues de la moelle osseuse.

7. Procédé selon la revendication 1, dans lequel l'agent cytotoxique comprend un agent cytotoxique chimique, un agent cytotoxique radiologique, une radiation ionisante, un agent cytotoxique biologique, un agent de guerre biologique, un agent de guerre chimique, un médicament présentant des effets secondaires indésirables chez les êtres humains, ou des combinaisons de ceux-ci.

8. Procédé selon la revendication 1, dans lequel le médicament de protection contre la cytotoxicité comprend de petits composés moléculaires, des peptides, des produits d'expression génique, des médicaments chimio-protecteurs, des médicaments radio-protecteurs, des médicaments antiviraux ou antibactériens, des médicaments angiogéniques ou anti-angiogéniques et des médicaments antiinflammatoires.

9. Procédé selon la revendication 8, dans lequel le médicament radio-protecteur présente une activité de prolifération anti-cellulaire synergiste dans les cellules tumorales de patients atteints du cancer lorsqu'il est administré à l'aide d'agents chimiothérapiques ou par radiation.

10. Procédé selon la revendication 8, dans lequel le médicament radio-protecteur est un médicament radio-protecteur non toxique comprenant des composés de sulfones d'aryle α,β insaturés.

11. Procédé selon la revendication 10, dans lequel le composé de sulfones d'aryle α,β insaturés comprend un sel de sodium E-4-carboxystyryl-4-chlorobenzylsulfone.

12. Procédé selon la revendication 8, dans lequel le médicament de protection contre la cytotoxicité exerce une radioprotection en empêchant les activités des gènes sensibles à la radiation, l'inhibition de l'apoptose, et la protection de l'ADN contre les dégâts provoqués par la radiation, ou une combinaison de ceux-ci .

13. Procédé selon la revendication 1, dans lequel les conditions de viabilité et de croissance des cellules cibles sont déterminées en analysant un ou plusieurs paramètre(s) ou marqueur(s) biologique(s) qui est (sont) régulé(s) à la hausse, régulé(s) à la baisse, ou exprimé(s) anormalement dans les cellules cibles lors de l'exposition à l'agent cytotoxique.

14. Procédé selon la revendication 13, dans lequel ledit (lesdits) un ou plusieurs paramètre(s) ou marqueur(s) biologique(s) est (sont) des molécules de peptide ou d'acide nucléique relatives à des chemins intracellulaires qui modulent une activité des gènes qui commandent une ou plusieurs réponse(s) cellulaire(s), des produits d'expression génique, des chemins biochimiques, des cascades de protéines, des récepteurs de cellules, des chemins et des molécules de signalisation de pré-apoptose, des fragments d'antigène ou d'anticorps, des agonistes ou des antagonistes de peptides, des marqueurs cytogénétiques ou une combinaison de ceux-ci.

15. Procédé selon la revendication 14, dans lequel ledit (lesdits) un ou plusieurs paramètre(s) comprend (comprennent) des marqueurs biologiques messagers de régulation de hématopoïèse.

16. Procédé selon la revendication 14, dans lequel les chemins intracellulaires sont impliqués dans l'apoptose, l'arrêt ou la réparation des cycles cellulaires, la prolifération, la sénescence ou la différentiation des cellules cibles.

17. Procédé selon la revendication 14, dans lequel les chemins intracellulaires appartiennent à la superfamille des protéines kinases activées par des facteurs mitogènes.

18. Procédé de criblage pour un médicament de protection contre la cytotoxicité, comprenant les étapes suivantes:
i) exposer a) des cellules cibles extraites chez un sujet non humain avant un traitement avec un composé de test de protection contre la cytotoxicité, et b) des cellules cibles extraites chez le sujet non humain après un traitement avec le composé de test de protection contre la cytotoxicité, à l'agent cytotoxique; et
ii) analyser et comparer un ou plusieurs paramètre(s) indicatif(s) de conditions de viabilité et de croissance dans les cellules cibles de l'étape i),
dans lequel une condition favorable de viabilité et de croissance des cellules cibles de l'étape ib) comparativement aux cellules cibles de l'étape ia) est indicative du potentiel du composé de test de protection contre la cytotoxicité pour son développement en tant que médicament de protection contre la cytotoxicité.

19. Utilisation d'un kit de test dans le procédé selon la revendication 1 ou la revendication 18, dans lequel le kit de test comprend des solutions et des dispositifs pour extraire des cellules cibles chez le sujet, un ou plusieurs composé (s) de test comme médicaments potentiels de protection contre la cytotoxicité, des supports et des plaques de culture de cellules et de tissus, un ou plusieurs agent(s) cytotoxique(s), des marqueurs biologiques et des essais pour analyser des paramètres indicatifs de conditions de viabilité et de croissance dans des cellules cibles, un ou plusieurs récipient(s) contenant les mélanges de réactifs nécessaires et les instructions pour l'utilisation de ceux-ci pour procéder à l'essai.
